# EUROPEAN PATENT SPECIFICATION

(11) **EP 3 845 073 B1**
(45) Date of publication and mention of the grant of the patent: **01.04.2026**
(21) Application number: 19854822.4
(22) Date of filing: 29.08.2019
(51) Int. Cl.: A23L 2/60, A23L 33/10, C07D 471/04, A23L 29/30, A23L 2/52, A23L 2/56

(54) **USE OF SWEETENERS FOR STABILIZING PYRROLOQUINOLINE QUINONE AND / OR SALTS THEREOF**
VERWENDUNG VON SÜSSUNGSMITTELN ZUR STABILISIERUNG VON PYRROLOCHINOCHINON UND/ODER DESSEN SALZEN
UTILISATION D'ÉDULCORANTS POUR STABILISER LA PYRROLOQUINOLINE QUINONE ET/OU SES SELS

(30) Priority: 30.08.2018 JP 2018162001
(43) Date of publication of application: 07.07.2021
(73) Proprietor: MITSUBISHI GAS CHEMICAL COMPANY, INC., Chiyoda-ku Tokyo 100-8324 (JP)
(72) Inventor: IKEMOTO, Kazuto, Niigata-shi, Niigata 950-3112 (JP); IMARUOKA, Satoko, Niigata-shi, Niigata 950-3112 (JP)
(74) Representative: Müller-Boré & Partner Patentanwälte PartG mbB
(86) International application number: PCT/JP2019/033887
(87) International publication number: WO 2020/045562

(56) References cited:
- EP-A1- 3 701 801
- WO-A1-2012/020767
- WO-A1-2012/157721
- WO-A1-2013/073642
- WO-A1-2018/180885
- JP-A- 2004 033 226
- JP-A- 2011 026 226
- JP-A- 2013 053 116
- JP-A- 2015 177 798
- JP-A- 2017 175 966
- JP-A- H01 156 903
- JP-A- H1 146 701
- JP-A- H11 123 069
- JP-A- S62 228 227
- US-A1- 2015 272 881
- US-A1- 2017 020 815

## Description

### Technical Field

The present invention widely relates to the use of a stabilizer for pyrroloquinoline quinone.

### Background Art

Pyrroloquinoline quinone has been known to have functionality such as brain function improvement, blood sugar level improvement, an antioxidant, and a life extension effect. Moreover, pyrroloquinoline quinone is a vitamin-like substance that also has a mitochondrial activation function.

Since pyrroloquinoline quinone not only has the various functionalities, but is also a highly safe component, it is contained in functional foods and supplements.

### Citation List

### Patent Literature

Patent Literature 1: Japanese Patent Laid-Open No. 2011-26226
Further, EP 3 701 801 A1 describes a pyrroloquinoline quinone-containing gummy candy and a method for producing the same;
US 2017/020815 A1 describes compositions and their use for allegedly improving human health, in particular, human brain health;
US 2015/272881 A describes a gel containing reduced pyrroloquinoline quinone and a method for producing the gel;
JP 2017-175966 A describes a pyrroloquinoline quinone-containing black tea-like beverage that does not require tea leaves;
JP H11-123069 A describes low-calorie acidic protein beverage and its manufacturing method;
JP 2015-177798 A describes a composition containing ascorbic acid and any one or more selected from guar gum, agar, erythritol, maltitol, chondroitin sulfate, and proteoglycan;
JP 2004-33226 A describes a low-calorie sweetening composition having a modified sweetness, a method for imparting such sweetness, and an acidic food or drink or pharmaceutical product and a neutral food or drink using the sweetening method;
JP H11-46701 A describes a low calorie syrup containing erythritol and Luo Han Guo glycoside;
WO 2012/157721 A1 describes a liposome composition, which enables the extension of the concentration range of pyrroloquinoline quinone in which the function thereof is exhibited, and a method for producing the same; and
WO 2012/020767 A1 describes a gel containing pyrroloquinoline quinone and a method for producing the gel.

### Summary of Invention

### Technical Problem

When manufacturing foods and supplements, sugars can be added thereto for various purposes. For example, Japanese Patent Laid-Open No. 2011-26226 describes that sucrose, a high-fructose corn syrup, etc. are used as sweeteners, and that there is no particular problem even when they coexist with pyrroloquinoline quinone.

However, as a result of the examination by the present inventors, it was newly found that sucrose, a high-fructose corn syrup, etc., generally contained in food, etc., can decrease pyrroloquinoline quinone with an elapse of time.

As a result of diligent examination to solve the above problems, the present inventors have found that by using a specific sweetening component, it is possible to provide pyrroloquinoline quinone having maintained stability, and thus have completed the present invention.

More specifically, the present invention is set out in the appended set of claims.

### Advantageous Effects of Invention

According to the present invention it is possible to store pyrroloquinoline quinone in a stable state for a long period of time even in solutions such as a beverage by containing a specific sweetening component. Description of Embodiments

In the first embodiment, the present invention provides the use of one or more sweetening components selected from the group consisting of erythritol, acesulfame potassium, and a reduced starch syrup to produce the stabilizer for pyrroloquinoline quinone and/or the salt thereof.

The reduced starch syrup may comprise any one of maltitol and/or sorbitol as a main component.

When erythritol is used as a sweetening component, a Luo Han Guo extract may be combined for use.

When the reduced starch syrup is used as a sweetening component, sucralose or a stevia extract may be combined for use.

The above sweetening components can also be used for the purpose of improving the original taste. The taste of a final product varies depending on the presence of components other than the pyrroloquinoline quinone and the active ingredients, however, the balance of taste can be adjusted by using a plurality of sweetening components.

The sweetening components used in the present invention has a sweetness equivalent to a concentration of 3 to 20% in terms of sucrose concentration, which is a combined value of sweetnesses of a reduced maltose starch syrup and the other sweetening components. More preferably, they have an amount of sweetness equivalent to a concentration of 5 to 15%. The reduced maltose starch syrup can be added at a concentration of 1 to 25%.

Pyrroloquinoline quinone used in the present invention is a substance having a structure represented by the following formula (1). This substance can also be present in the form of salt.

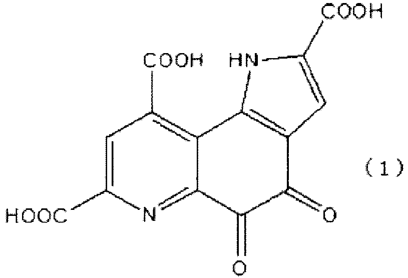

The "salt of pyrroloquinoline quinone" used in the present invention includes an alkali metal salt, an alkaline earth metal salt, and an ammonium salt, of pyrroloquinoline quinone, however, the alkali metal salt is preferred.

The alkali metal salt of pyrroloquinoline quinone used in the present invention includes salts of sodium, potassium, lithium, cesium, rubidium, etc. Preferably, the sodium salt or the potassium salt is more preferable in terms of easy availability thereof. Pyrroloquinoline quinone may be an alkali metal salt of pyrroloquinoline quinone, substituted with 1 to 3 alkali metal atoms, and may be any of a monoalkali metal salt, a dialkali metal salt, or a trialkali metal salt, however, the dialkali metal salt is preferred. The alkali metal salt of pyrroloquinoline quinone is particularly preferably disodium salt or dipotassium salt.

The pyrroloquinoline quinone and/or the salt thereof used in the present invention may be commercially available products, or they can be produced by a known method.

When the final product is a beverage, the pyrroloquinoline quinone and/or the salt thereof is contemplated to have a concentration of, for example, about 0.01 to 1 g/L, and preferably 0.02 to 0.5 g/L. Although not intended to be limited, the amount of active ingredients required for stabilization of pyrroloquinoline quinone in such a concentration is an amount equivalent to 3 to 20% by weight in terms of sucrose concentration. For example, when the sweetness of sucrose is 100, the sweetness of erythritol is 75 to 85, and therefore the amount required for stabilization of pyrroloquinoline quinone is 3.5 to 26.7% by weight. Moreover, the amount of acesulfame potassium which has 200 to 700 times the sweetness of sucrose, required to stabilize pyrroloquinoline quinone is 0.004 to 0.1% by weight.

The content of pyrroloquinoline quinone can be measured by an analytical method suitable for the conditions of the measurement sample among the commonly known analytical methods for pyrroloquinoline quinone. Specifically, it is possible to analyze it by the liquid chromatography described in Examples to be described later. Incidentally, at the time of measurement, an appropriate treatment may be carried out as necessary, such as freeze-drying a sample to match the detection range of the apparatus, and removing impurities in the sample to match the separating power of the apparatus.

In the second embodiment, the present invention provides using the stabilizer, according to the first embodiment, in a composition.

The form of the composition is not particularly limited provided that it can contain the stabilizer, however, the composition is contemplated to be provided as food or drink. The stabilizer can be suitably used in beverages, in particular, acidic beverages, among food or drink. The form of the beverage can be exemplified as a sports beverage, a fruit beverage, a tea beverage, a coffee beverage, a dairy beverage, a carbonated beverage, a functional beverage, an energy drink, etc., however, is not limited thereto.

The acidic beverage contemplated in the present invention refers to a beverage using an acidulant and having a pH range of 2 to 5.5. In the case of a sports beverage, it often provides cool sensation by rendering a pH acidic, which can be adjusted to a pH of 1 to 4.

The acidulant used to adjust a pH is not particularly limited provided that it is a substance that can be added to a food. Citric acid, etc., are exemplified as such examples. Ascorbic acid is also commonly used as a pH adjusting acidulant, however may reduce the stability of pyrroloquinoline quinone.

The acidulant in the acidic beverage of the present invention can be used in an amount of 0.0001 g/L to 15 g/L and more preferably 0.01 to 4 g/L.

Various additives generally used in food or drink can be added to the food or drink as a final product provided that the stabilizing effect of the sweetening component is not impaired. Examples of such additives include a sweetener, an acidulant, a preservative, a pigment, an antioxidant, and a fragrance. Moreover, food components such as a fruit juice, a coffee extract, a tea leaf extract, and a milk component may be added depending on the purpose. The amount of the additive can be appropriately determined by the person skilled in the art by comparatively weighing the desired effect and the influence on the stability.

Naturally, the sweetening component described above also serves as a sweetener due to its inherent properties, however, the food or drink may further comprise another sweetener provided the stabilizing effect of the sweetening component is not impaired.

Examples of the fragrance include a peach essence, a vanilla essence, a strawberry essence, an apple essence, a lemon essence, and a drink fragrance. By adding these, the taste of food or drink can be rendered more favorable.

Further examples of the additives include fruit juices, plant extracts, carbon dioxide, vitamins, minerals, pigments, emulsifiers, seasonings, and quality stabilizers. The content of these additives can also be appropriately set within a range that does not impair the object of the present invention.

The beverage is contemplated to be a non-alcoholic beverage in consideration of the functionality of pyrroloquinoline quinone, however it may contain alcohol.

The non-alcoholic beverage refers to a beverage containing less than 1% by mass of ethanol. For example, a carbonated beverage, a fruit juice, a vegetable juice, a sports beverage, an isotonic beverage, enhanced water, bottled water, a near water drink, a coffee beverage, a tea beverage, a beer taste beverage, an energy drink, and a beauty drink are included.

Examples of the alcoholic beverage include beer, wine, sake, plum wine, low-malt beer, whiskey, brandy, shochu (distilled spirits), rum, gin, and liqueurs.

The beverages can be sterilized by heating depending on the aspect of the product. The method of heat sterilization is contemplated to meet the conditions specified in the applicable regulations (Food Sanitation Law in Japan), and includes, for example, a retort sterilization method, a high temperature short time sterilization method (HTST method), an ultrahigh temperature sterilization method (UHT method), and a post-filling sterilization method (pasteurization).

Moreover, the method of heat sterilization can be appropriately selected, and, for example, when the entire container such as a metal can or a bottle after being filled with a beverage, can be sterilized by heating (for example, 60 to 140°C for 1 to 60 minutes), the retort sterilization and the post-filling sterilization method (pasteurization) can be employed. In the case of the post-filling sterilization method (pasteurization), heat sterilization can be carried out, for example, at 65°C for 1 to 60 minutes, preferably 65°C for 5 to 30 minutes, and more preferably 65°C for 10 to 20 minutes.

For PET bottles that cannot be subjected to the retort sterilization, aseptic filling, hot pack filling, etc. can be employed, in which a beverage is sterilized by heating in advance under the same sterilization conditions as above (for example, at 65 to 140°C for 0.1 seconds to 30 minutes, preferably at 70 to 125°C for 1 second to 25 minutes, and more preferably at 75 to 120°C for 10 seconds to 20 minutes), and then it fills a container which has been subjected to sterilization in a sterile environment.

The beverage can be provided as a packaged acidic beverage by filling an ordinary packaging container such as a molded container (so-called PET bottle) containing polyethylene terephthalate as a main component, a metal can, or a bottle. Small containers that are 50 to 200 mL containers are preferred.

### Examples

BioPQQ manufactured by MITSUBISHI GAS CHEMICAL COMPANY, INC. was used as the pyrroloquinoline quinone disodium salt used in the present invention. The reduced maltose starch syrup, MU-75 (solid content of 75%) manufactured by UENO FOOD TECHNO INDUSTRY, LTD. was used. This is a maltitol syrup by reduction of maltose obtained by saccharifying starch. If this product has a solid sugar content of 100, it contains 4 or less of monosaccharide, 73 or more of disaccharide, trisaccharide in the range of 14 to 20, and oligosaccharide of tetrasaccharide or more in the range of 5 to 11. The reagent manufactured by Wako Pure Chemical Industries was used unless otherwise specified. The high-fructose corn syrup used was a high-fructose corn syrup manufactured by Oji Cornstarch Co., Ltd. The composition indicates the weight of the product used, and the water content and purity are not corrected. Sodium citrate for production of trisodium citrate was used.
Analysis conditions of pyrroloquinoline quinone disodium salt
HPLC conditions:
   Shimadzu LC-2010
   Column: YMC-Pack ODS-A
   Detection wavelength: 259 nm
   Mobile phase: 30 mM acetic acid - 70 mM ammonium acetate
   Column temperature: 40°C

Prepared samples were subjected to a sensory test by employing one researcher in MITSUBISHI GAS CHEMICAL COMPANY, INC. as a panelist.

### Evaluation score of taste

5: Very delicious
4: Deep and delicious
3: Monotonous
2: Slightly pungent
1: Strongly pungent

### (Example 1) Combination of reduced starch syrup, sugar alcohol, and high-sweetness sweetener

A beverage was prepared by mixing 5 g of a reduced maltose starch syrup, 2 g of erythritol, 10 mg of acesulfame K, 60 mg of citric acid, 40 mg of sodium citrate, 22 mg of BioPQQ, 100 mg of a strawberry essence (manufactured by Golden Kelly Pat. Flavor Co., Ltd.), and the balance water such that the mixture had a total volume of 50 mL. The evaluation score of taste was 5.

### (Accelerated storage test)

The sample of Example 1 was stored under the conditions of 60°C for 2 weeks, which are equivalent to those of room temperature for 2 years. When the stored sample was subjected to HPLC, 97% of BioPQQ was recovered. No precipitate was found in the beverage.

### (Example 2) Combination of reduced starch syrup, sugar alcohol, and high-sweetness sweetener

A beverage was prepared in the same manner as in Example 1 except that an equivalent amount of water was added instead of the strawberry essence, and a sensory test and an accelerated storage test were carried out.

The evaluation score of the taste of the beverage of Example 2 was 4. When the sample was subjected to HPLC after an accelerated storage test equivalent to a storage test at room temperature for 2 years, 98% of BioPQQ was recovered. No precipitate was found in the beverage.

### (Example 3)

A beverage was prepared in the same manner as in Example 1 except that 100 mg of a lemon essence (manufactured by Golden Kelly Pat. Flavor Co., Ltd.) was added instead of 100 mg of the strawberry essence, and a sensory test and an accelerated storage test at 60°C for 1 week (equivalent to a storage test for 1 year) were carried out.

When the sample was subjected to HPLC after an accelerated storage test equivalent to a storage test at room temperature for 1 year, 99% of BioPQQ was recovered. No precipitate was found in the beverage. The evaluation score of the taste of the beverage of Example 3 was 4.

### (Comparative Examples 1 and 2)

Beverages were each prepared in the same manner as in Example 2 except that the sweetening components used were changed from 5 g of the reduced maltose starch syrup, 2 g of erythritol, and 10 mg of acesulfame K to those as described in the table below, and a sensory test and an accelerated storage test at 60°C for 1 week (equivalent to a storage test at room temperature for 1 year) were carried out. The results are shown in the table below.

**[Table 1]**

| | Sweetener | Evaluation score of taste | BioPQQ recovery (%) |
|---|---|---|---|
| Comparative Example 1 | Sucrose 4 g | 4 | 89 |
| Comparative Example 2 | High-fructose corn syrup from Oji 5 g | 4 | 61 |

As a result of replacing the combination of the reduced maltose starch syrup, erythritol, and acesulfame with sucrose alone or a high-fructose corn syrup alone, the recovery of pyrroloquinoline quinone was decreased. Moreover, the recovery of pyrroloquinoline quinone was also decreased even when aspartame alone was used, although no results are shown.

### (Examples 4 to 10) Change of fragrance

Beverages were each prepared in the same manner as in Example 1 except that the fragrance described in the table below was added instead of the strawberry essence, and a sensory test and an accelerated storage test were carried out. The conditions for the accelerated storage test were 60°C for 2 weeks for the samples of Examples 4 to 6 and 60°C for 1 week for the samples of Examples 7 to 10. The results are shown below.

**[Table 2]**

| | Fragrance | Evaluation score of taste | BioPQQ recovery (%) |
|---|---|---|---|
| Example 3 | Apple natural fragrance 100 mg | 5 | 100 |
| Example 4 | Crushed grapefruit fragrance 100 mg | 5 | 97 |
| Example 5 | Energy drink flavor 100 mg | 5 | 96 |
| Example 6 | Apple natural fragrance 200 mg | 5 | 98 |
| Example 7 | Crushed grapefruit fragrance 200 mg | 5 | 100 |
| Example 8 | Energy drink flavor 200 mg | 5 | 97 |
| Example 9 | Strawberry essence 200 mg | 5 | 97 |

Beverages with excellent stability and taste were obtained. Moreover, no precipitate was found in all the samples.

### (Examples 11 to 14) Reduced starch syrup alone or combination with high-sweetness sweetener

Beverages were each prepared in the same manner as in Example 1 except that the sweetening components used were changed from 5 g of the reduced maltose starch syrup, 2 g of erythritol, and 10 mg of acesulfame K to those as described in the table below, and a sensory test and an accelerated storage test at 60°C for 1 week (equivalent to a storage test for 1 year) were carried out.

**[Table 3]**

| | Sweetener | BioPQQ recovery (%) | Evaluation of taste |
|---|---|---|---|
| Example 11 | Reduced maltose starch syrup 5 g, Acesulfame K 10 mg | 97 | 5 |
| Example 12 | Reduced maltose starch syrup 5 g, Sucralose 10 mg | 99 | 5 |
| Example 13 | Reduced maltose starch syrup 5 g, Stevia 10 mg | 98 | 5 |
| Example 14 | Reduced maltose starch syrup 5 g | 99 | 4 |

### (Example 15) Combination of sugar alcohol and high-sweetness sweetener

A beverage was prepared in the same manner as in Example 1 except that the sweetening components used were replaced from 5 g of a reduced maltose starch syrup, 2 g of erythritol, and 10 mg of acesulfame K to 2.5 g of erythritol and 10 mg of acesulfame K, and a sensory test and an accelerated storage test at 60°C for 2 weeks (equivalent to a storage test at room temperature for 2 years) were carried out. The evaluation score of taste was 4, and the recovery of BioPQQ was 100%. There was no precipitate.

### (Example 16)

A beverage was prepared in the same manner as in Example 10 except that the sweetening components used were changed from 5 g of the reduced maltose starch syrup, 2 g of erythritol, and 10 mg of acesulfame K to 20 mg of acesulfame K, and a sensory test and an accelerated storage test at 60°C for 2 weeks (equivalent to a storage test at room temperature for 2 years) were carried out. The BioPQQ recovery was 100%. The evaluation score of the taste was 3. Precipitates were found in the beverage. As described above, when the sweetener was acesulfame K alone, the storage stability of pyrroloquinoline quinone was high, however, precipitation occurred, and the taste became monotonous.

### (Example 17) Combination of sugar alcohol and high-sweetness sweetener

A beverage was prepared by mixing 11 g of Lacanto (erythritol/Luo Han Guo extract manufactured by Saraya Co., Ltd.), 120 mg of citric acid, 80 mg of sodium citrate, 22 mg of BioPQQ, and the balance water such that the mixture had a total volume of 100 mL. The evaluation score of the taste was 4, and the BioPQQ recovery after 1 week at 60°C was 100%.

### (Examples 18 to 20) Change of reduced starch syrup

Beverages were each prepared in the same manner as in Example 1 except that the reduced starch syrup described in the table below was added in place of the reduced maltose starch syrup (both manufactured by B Food Science Co., Ltd.), and a sensory test and an accelerated storage test at 60°C for 1 week (equivalent to a storage test for 1 year) were carried out. The main components of all the reduced starch syrup used are monosaccharide sorbitol and disaccharide maltitol. When the solid content is 100, SE 600 is composed of monosaccharide in the range of 40 to 50, disaccharide in the range of 40 to 50, trisaccharide in the range of 8 to 13, tetrasaccharide in the range of 1 to 5, and pentose or more in the range of 1 to 5. SweetG2 is composed of monosaccharide in the range of 1 to 6, disaccharide in the range of 70 to 77, trisaccharide in the range of 10 to 20, tetrasaccharide in the range of 1 to 6, and pentose or more in the range of 1 to 10. SweetP EM is composed of monosaccharide in the range of 40 to 50, disaccharide in the range of 45 to 55, trisaccharide in the range of 1 to 5, tetrasaccharide in the range of 0 to 3, and pentose or more in the range of 0 to 3.

**[Table 4]**

| | Sweetening component | BioPQQ recovery (%) | Evaluation of taste |
|---|---|---|---|
| Example 18 | SE 600 5 g | 99 | 4 |
| Example 19 | SweetG2 5 g | 100 | 4 |
| Example 20 | SweetP EM 5 g | 97 | 4 |

### (Examples 21 to 25) Sugar alcohol alone, high-sweetness sweetener alone, combination of sugar alcohol and high-sweetness sweetener

Beverages were each prepared by mixing the sweetening components described in the table below, 70 mg of citric acid, 30 mg of sodium citrate, 20 mg of BioPQQ, and the balance water such that the mixture had a total volume of 100 mL. The prepared beverages were stored at 60°C for a predetermined period and subjected to HPLC analysis. The results are shown below.

**[Table 5]**

| | Sweetening component | BioPQQ recovery (%) | Storage period |
|---|---|---|---|
| Example 21 | Erythritol 11 g | 100 | 1 week |
| Example 22 | Erythritol 11 g | 99 | 2 weeks |
| Example 23 | Acesulfame K 0.04 g | 99 | 1 week |
| Example 24 | Lakanto Luo Han Guo extract + Erythritol 6 g | 95 | 1 week |
| Example 25 | Lakanto Luo Han Guo extract + Erythritol 6 g | 93 | 2 weeks |
| Comparative Example 5 | Sucrose 8 g | 85 | 1 week |

Erythritol and Lacanto showed a high stabilizing effect, and among these, erythritol was stable even when stored under the conditions equivalent to those of room temperature for 2 years.

## Claims

1. Use of one or more sweetening components selected from the group consisting of erythritol, acesulfame potassium, and a reduced starch syrup as a stabilizer for pyrroloquinoline quinone and/or a salt thereof.

2. The use according to claim 1, wherein the reduced starch syrup comprises maltitol and/or sorbitol as main components.

3. The use according to claim 1, wherein the sweetening component is erythritol and the stabilizer further comprises a Luo Han Guo extract.

4. The use according to claim 1 or 2, wherein the sweetening component is a reduced starch syrup, and the stabilizer further comprises sucralose and/or a stevia extract.

5. The use according to any one of claims 1 to 4, wherein the salt of pyrroloquinoline quinone is pyrroloquinoline quinone disodium salt.

6. The use according to any one of claims 1 to 5, wherein the stabilizer is comprised in a composition.

7. The use according to claim 6, wherein the composition is in the form of food or drink.

8. The use according to claim 7, wherein the composition is in the form of an acidic beverage.

## Patentansprüche

1. Verwendung eines oder mehrerer Süßungsmittel, ausgewählt aus der Gruppe, bestehend aus Erythrit, Acesulfam-Kalium und einem stärkereduzierten Sirup, als Stabilisator für Pyrrolochinolinchinon und/oder ein Salz davon.

2. Verwendung nach Anspruch 1, wobei der reduzierte Stärkesirup Maltit und/oder Sorbit als Hauptbestandteile enthält.

3. Verwendung nach Anspruch 1, wobei das Süßungsmittel Erythrit ist und der Stabilisator weiter einen Luo Han Guo-Extrakt umfasst.

4. Verwendung nach Anspruch 1 oder 2, wobei das Süßungsmittel ein stärkereduzierter Sirup ist und der Stabilisator weiter Sucralose und/oder einen Stevia-Extrakt umfasst.

5. Verwendung nach einem der Ansprüche 1 bis 4, wobei es sich bei dem Salz des Pyrrolochinolinchinons um das Dinatriumsalz des Pyrrolochinolinchinons handelt.

6. Verwendung nach einem der Ansprüche 1 bis 5, wobei der Stabilisator in einer Zusammensetzung enthalten ist.

7. Verwendung nach Anspruch 6, wobei die Zusammensetzung in der Form eines Lebensmittels oder Getränks vorliegt.

8. Verwendung nach Anspruch 7, wobei die Zusammensetzung in der Form eines sauren Getränks vorliegt.

## Revendications

1. Utilisation d'un ou plusieurs composants édulcorants sélectionnés parmi le groupe constitué de l'érythritol, de l'acésulfame de potassium, et d'un sirop d'amidon réduit en tant que stabilisant pour de la pyrroloquinoline quinone et/ou un sel de celle-ci.

2. Utilisation selon la revendication 1, dans laquelle le sirop d'amidon réduit comprend du maltitol et/ou sorbitol en tant que composants principaux.

3. Utilisation selon la revendication 1, dans laquelle le composant édulcorant est l'érythritol et le stabilisant comprend en outre un extrait de Luo Han Guo.

4. Utilisation selon la revendication 1 ou 2, dans laquelle le composant édulcorant est un sirop d'amidon réduit, et le stabilisant comprend en outre du sucralose et/ou un extrait de stévia.

5. Utilisation selon l'une quelconque des revendications 1 à 4, dans laquelle le sel de pyrroloquinoline quinone est le sel disodique de pyrroloquinoline quinone.

6. Utilisation selon l'une quelconque des revendications 1 à 5, dans laquelle le stabilisant est compris dans une composition.

7. Utilisation selon la revendication 6, dans laquelle la composition est sous la forme d'aliment ou de boisson.

8. Utilisation selon la revendication 7, dans laquelle la composition est sous la forme d'une boisson acide.
